# EUROPÄISCHE PATENTSCHRIFT

(11) **EP 1 474 940 B1**
(45) Veröffentlichungstag und Bekanntmachung des Hinweises auf die Patenterteilung: **09.05.2007**
(21) Anmeldenummer: 03739482.2
(22) Anmeldetag: 12.02.2003
(51) Int. Cl.: H04Q 7/32, A61B 5/00

(54) **ZENTRALEINHEIT, NEBENEINHEIT UND VERFAHREN ZUM KOMMUNIZIEREN**
CENTRAL PROCESSING UNIT, AUXILIARY UNIT AND METHOD FOR COMMUNICATION
UNITE CENTRALE, UNITE SECONDAIRE ET PROCEDE DE COMMUNICATION ENTRE CES DERNIERES

(30) Priorität: 12.02.2002 DE 10205710
(43) Veröffentlichungstag der Anmeldung: 10.11.2004
(73) Patentinhaber: Fraunhofer-Gesellschaft zur Förderung der angewandten Forschung e.V., 80686 München (DE)
(72) Erfinder: HUPP, Jürgen, 90425 Nürnberg (DE); GEHRMANN, Volker, 91092 Erlangen (DE); MACK, Doris, 90419 Nürnberg (DE); STURM, Roland, 90491 Nürnberg (DE)
(74) Vertreter: Zinkler, Franz
(86) Internationale Anmeldenummer: PCT/EP2003/001385
(87) Internationale Veröffentlichungsnummer: WO 2003/069933

(56) Entgegenhaltungen:
- EP-A- 1 158 685
- WO-A-01/69859
- DE-A- 19 929 474
- US-A- 5 767 791
- "Technical Specification, Broadband Radio Access Networks (BRAN); HIPERLAN Type 2; Data Link Control (DLC) Layer; Part 2: Radio Link Control (RLC)" ETSI STANDARD ETSI TS 101 761-2 V1.3.1, Januar 2002 (2002-01), XP002374892
- "Technical Specification, Broadband Radio Access Networks (BRAN); HIPERLAN Type 2; Data Link Control (DLC) Layer; Part 1: Basic DataTransport Functions" ETSI STANDARD ETSI 101 761-1 V.1.3.1, Dezember 2001 (2001-12), XP002374893

## Beschreibung

Die vorliegende Erfindung bezieht sich auf eine Kommunikation zwischen einer Zentraleinheit und einer Nebeneinheit, auf eine dafür vorgesehene Zentraleinheit und eine dafür vorgesehene Nebeneinheit und insbesondere auf eine Kommunikation mittels eines körpernahen Funknetzwerks (BAN; BAN Body Area Network).

Bei einem körpernahen Funknetzwerk kommunizieren dezentrale Sensoren zur Erfassung von Vitalparametern, wie z. B. Temperatur, Blutdruck, EKG-Ableitung, etc. mit einer Zentraleinheit.

In einem körpernahen Funknetzwerk sollen mehrere Sensoreinheiten existieren. In einer Zentralstation soll eine Anzeige, Verarbeitung und Weiterleitung der Daten drahtlos oder leitungsgebunden erfolgen.

Insbesondere bei körpernahen Funknetzwerken, und allgemein bei beliebigen Funknetzwerken besteht der Wunsch, daß das System und insbesondere die entfernt angeordnete Sensoreinheit einen möglichst geringen Stromverbrauch haben soll.

Hierfür existieren im Stand der Technik verschiedene Möglichkeiten. Bei DECT-Systemen (DECT = Digital Enhanced Cordless Telephone) existieren lediglich lokal gesteuerte Stromspar-Modi. Dies bedeutet, daß sich ein DECT-Mobilteil bei lokaler Nichtaktivität in einen Stromsparmodus versetzt. Aufgrund der Tatsache, daß DECT verbindungsorientiert arbeitet, ist eine Funkverbindung mit Sende/Empfangsvorgängen auch notwendig, wenn keine Nutzdaten übertragen werden. Dies bedeutet, mit anderen Worten, daß der Sender/Empfänger des DECT-Mobilteils bei Bestehen einer Verbindung nicht in einen Stromspar-Modus versetzt werden kann, da er auch im Falle einer Nichtaktivität beispielsweise eines Körpersensors, dennoch Signalisierungsaufgaben zu erfüllen hat. Eine lokale Nichtakitvität ist nur dann gegeben, wenn die Verbindung unterbrochen ist.

Im Falle der WLAN-Technik (WLAN = Wireless Local Area Network) wird eine paketbasierte Nachrichtenübertragung verwendet. Dies bedeutet, daß WLAN-Empfangsgeräte sehr lange im Empfangszustand sein müssen, da die Empfangspakete beliebig eintreffen können.

Bei Hiper-LAN 2 existiert ein eigener Control-Channel, durch den ein Master signalisiert, wann ein Slave-Gerät Sende- und/oder Empfangsphasen haben soll.

Bei Bluetooth verfügen Slaves über Stromsparmodi, in denen ihre Aktivität verringert wird. Nur ein einzelnes Ansprechen der Slaves und ein Versetzen der einzelnen Slaves in den Stromsparmodus über einen logischen Signalisierungskanal ist möglich. Nachdem Bluetooth kein verbindungsorientiertes System ist, müssen Slaves zunächst Datenpakete empfangen, um dann feststellen zu können, ob ein Datenpaket für einen speziellen Slave bestimmt war, um daraus zu ermitteln, ob der Slave in einen Stromsparmodus gehen darf oder nicht.

Nachteilig an allen beschriebenen Konzepten ist, daß die Signalisierung von Schlafmodusinformationen für verschiedene Nebeneinheiten problematisch ist, da Slaves entweder nur einzeln angesprochen werden können oder andererseits aufgrund der verbindungsorientierten Datenübertragung und aufgrund der paketgebundenen Datenübertragung dennoch eine beachtliche Sende/Empfangsaktivität zeigen müssen, um überhaupt feststellen zu können, ob sie in einen Schlafmodus gehen sollen oder nicht. Im Falle eines verbindungsorientierten Netzwerks ist eine vollständige Empfängerabschaltung nicht möglich, da dies z. B. von einer DECT-Zentraleinheit als Abmeldung interpretiert werden würde.

Darüber hinaus existiert ein Problem dahingehend, daß, wie im Falle von Bluetooth, ein Versetzen eines Slave-Geräts in einen Stromsparmodus, also eine Abschaltung seines Empfängers dazu führt, daß das Slave-Gerät im Stromsparmodus nicht in der Lage ist, eine Notfallmeldung abzusetzen, da die Zentraleinheit für keinen Empfang von diesem Slave-Gerät vorbereitet ist. Insbesondere für körpernahe Funknetzwerke bei denen Sensoren und Aktoren zur Erfassung bzw. Beeinflussung von Vitalparametern vorgesehen sind, ist eine solche Notfallfunktion - trotz aktiviertem Schlafmodus - nicht möglich.

Ein weiterer Nachteil beispielsweise von Bluetooth ist, daß ein eigener Signalisierungskanal für jedes Slave-Gerät erforderlich ist, wobei ein Slave-Gerät zumindest einen Downlink, also eine Sendung von der Zentraleinheit zum Slave-Gerät empfangen muß, um festzustellen, ob dieses Paket auch für das tatsächlich vorliegende Nebengerät gedacht war. Alle Slave-Geräte müssen daher alle Datenpakete des Signalisierungskanals empfangen, obgleich diese Datenpakete nur an einzelne Slave-Geräte gerichtet sind.

Die EP 0 615 364 A1 offenbart ein drahtloses Datenkommunikationssystem mit einer Leistungssparfunktion. Eine Zentraleinheit sendet in regelmäßigen Abständen eine sogenannte TIM-Nachricht und optional zwischen zwei TIM-Nachrichten Daten-Interrupt-Signale, die einen Sendebetrieb von der Zentraleinheit zu einer Station signalisieren. Wenn eine Station in einer TIM-Nachricht nicht angesprochen wird, so folgert sie, dass sie in diesem TIM-Intervall in einen Leistungssparmodus gehen kann. Erhält eine Station eine Nachricht, dass sie in einem TIM-Intervall in den Schlafmodus gehen kann, so schaltet sie sich nach einem Empfang des TIM-Signals ab und aktiviert sich - ansprechend auf einen in ihr enthaltenen entsprechend eingestellten Timer - wieder kurz vor dem Empfang eines folgenden Signals, um dort Informationen zu erhalten, ob sie in dem nächsten TIM-Intervall wieder in den Leistungssparmodus gehen kann oder nicht.

Das US-Patent Nr. 5,767,791 zeigt eine Niederleistungsschaltung und ein Verfahren zum Schaffen einer schnellen Frequenzverriegelung in einem drahtlosen Kommunikationsgerät. Insbesondere arbeitet eine Zentraleinheit mit mehreren Nebeneinheiten, wobei die Nebeneinheiten von einem Patienten getragen werden und ihre Informationen zur Zentraleinheit senden. Die Kommunikation findet innerhalb einer Überrahmen-Struktur statt, wobei ein Multirahmen zwei Downlink-Rahmen umfaßt, und mehrere Uplink-Rahmen, sowie einen speziellen Netzanforderungsrahmen (Net Req.). Jede Nebeneinheit geht in den Schlafmodus, sobald sie ihre Nachrichtenübertragung beendet hat.

Die WO 01/69859 A1 offenbart eine Weiterentwicklung des Hiperlan Type 2 Standards, bei dem die Nebeneinheiten über einen eigenen Kanal der Zentraleinheit mitteilen, daß sie in den Schlafmodus gehen möchten. Ferner schlagen die Nebeneinheiten eine Schlafmodus-Dauer für das Schlafintervall vor. Die Zentraleinheit erhält das Schlafmodus-Anforderungssignal, entscheidet die Startzeit und die Schlafdauer und sendet dann ein Schlafreservierungssignal zu der Nebeneinheit, das die Startzeit anzeigt, zu der die Nebeneinheit in den Schlafmodus gehen soll. Ferner sendet die Zentraleinheit die Schlafdauer oder Zeitdauer zu der Nebeneinheit, die sie im Schlafmodus bleiben soll, bevor die Nebeneinheit wieder aufwachen soll, um den BCCH Abschnitt eines MAC-Frames zu überwachen, um herauszufinden, ob es Daten für die Nebeneinheit gibt.

Die Aufgabe der vorliegenden Erfindung besteht darin, ein effizienteres Konzept zum Kommunizieren zwischen einer Zentraleinheit und einer Nebeneinheit zu schaffen.

Diese Aufgabe wird durch eine Zentraleinheit nach Patentanspruch 1, eine Nebeneinheit nach Patentanspruch 17, ein Verfahren zum Kommunizieren zwischen einer Zentraleinheit und einer Nebeneinheit nach Patentanspruch 25 oder ein Verfahren zum Kommunizieren zwischen einer Nebeneinheit und einer Zentraleinheit nach Patentanspruch 26 gelöst.

Der vorliegenden Erfindung liegt die Erkenntnis zugrunde, daß eine effiziente und erhebliche Stromeinsparungen ermöglichende Signalisierung dadurch erfolgen kann, daß eine Multirahmen-Struktur verwendet wird, wobei jeder Multirahmen einen Steuerrahmen und zumindest einen Nutzrahmen aufweist. Die von der Zentraleinheit abgesendeten Daten sind somit in Multirahmen im Sinne eines Zeitmultiplex strukturiert, so daß ein Multirahmen mit einem Steuerrahmen beginnt, in dem jeder vorhandenen Nebeneinheit Stromsparmodusinformationen zugewiesen werden können, wenn diese Nebeneinheiten in einen Stromsparmodus innerhalb des aktuellen oder eines folgenden Multirahmens gehen sollen. Ferner wird in dem Steuerrahmen signalisiert, welcher Nutzrahmen des Multirahmens welcher Nebeneinheit für welche Kommunikation, z. B. eine Uplink-Kommunikation, d. h. von der Nebeneinheit zur Zentraleinheit, oder eine Downlink-Kommunikation, d. h. von der Zentraleinheit zur Nebeneinheit, vorgesehen ist.

Vorzugsweise kann zur aktiven Übertragungskapazitätssteuerung in dem Steuerrahmen signalisiert werden, daß eine Zentraleinheit innerhalb des betrachteten Multirahmens mehr als einen Uplink-Nutzrahmen zugewiesen bekommt, um eine höhere Datenmenge als eine andere Nebeneinheit zur Zentraleinheit übertragen zu können.

Der Multirahmen ist ferner so aufgebaut, daß er einen Freirahmen umfaßt, d. h. einen Rahmen, in dem der Empfänger der Zentraleinheit in Empfangsbereitschaft ist, und der keiner Nebeneinheit zur Nutzdatenübertragung zugewiesen ist. Dieser Freirahmen oder Random-Frame dient dazu, daß sich neue Nebeneinheiten selbständig anmelden können. Darüber hinaus erlaubt dieser Freirahmen, daß eine Nebeneinheit trotz der Tatsache, daß sie in den Stromsparmodus "geschickt" worden ist, eine Notfallmeldung zur Zentraleinheit senden kann, wenn bestimmte - lokal in der Nebeneinheit definierte - Notfallbedingungen erfüllt sind.

Die vorliegende Erfindung ist dahingehend vorteilhaft, daß eine transparente und effizienten Netzaktivitätssteuerung dadurch möglich wird, daß eine Multirahmenstruktur verwendet wird, in der ein Steuerrahmen einer Mehrzahl von Nutzrahmen vorausgeht, wobei ferner vorzugsweise ein Freirahmen in jedem oder zumindest einigen Multirahmen der Folge von Multirahmen vorhanden ist, um eine Empfangsbereitschaft der Zentraleinheit für Notfallmeldungen einerseits und um Neuanmeldungen von Netzeinheiten andererseits mit überschaubarem Aufwand ermöglichen zu können.

Ein weiterer Vorteil der vorliegenden Erfindung besteht darin, daß der Steuerrahmen derart geartet ist, daß er eine flexible Netzressourcenverteilung von Multirahmen zu Multirahmen ermöglicht, indem von Multirahmen zu Multirahmen mehr oder weniger Nutzrahmen einer Nebeneinheit zugewiesen werden.

Ein weiterer Vorteil der vorliegenden Erfindung besteht darin, daß eine effiziente Signalisierung von Schlafmodusinformationen möglich ist. Sämtliche Nebeneinheiten, die nicht im Schlafmodus sind, müssen lediglich den Steuerrahmen empfangen. Stellen sie fest, daß sie in einen Schlafmodus dürfen, so kann sofort, also vor dem Empfang weiterer Nutzrahmen, eine Deaktivierung des Empfängers und ggf. auch des Senders stattfinden, und zwar für eine Zeitdauer, die in dem Steuerrahmen vorzugsweise als Anzahl von aufeinanderfolgenden Multirahmen signalisiert wird.

Ein weiterer Vorteil der vorliegenden Erfindung besteht darin, daß neben der Tatsache, daß eine schnelle und wenig aufwendige Schlafmodus-Aktivierung möglich ist, auch im Sende/Empfangs-Modus leistungssparend gefahren werden kann, da ein Sender nur in dem Nutzrahmen des Multirahmens aktiv zu sein braucht, in dem die Nebeneinheit senden soll, und da natürlich auch der Empfänger der Nebeneinheit nur in dem Nutzrahmen empfangsbereit zu sein braucht, und daher Strom verbraucht, in dem die Zentraleinheit Daten zu der betrachteten Nebeneinheit senden möchte. Welcher Rahmen dies ist, wird durch den Steuerrahmen vollständig signalisiert.

Ein weiterer Vorteil der vorliegenden Erfindung besteht darin, daß Nebeneinheiten, die z. B. für eine bestimmte Anzahl von Multirahmen in den Schlafmodus versetzt worden sind, in der Zwischenzeit, also wenn sie im Schlafmodus sind, keine Empfangsbereitschaft und damit keinen hierfür erforderlichen Strom aufwenden müssen, da die für einen Betrieb nach der Schlafmoduszeit erforderlichen Informationen in dem dann folgenden Multirahmen, also dem Multirahmen nach der Schlafmoduszeit enthalten sind, und zwar im Steuerrahmen des dann auftretenden Multirahmens.

Ein weiterer Vorteil der vorliegenden Erfindung besteht darin, daß in dem Steuerrahmen für einen Multirahmen auch Schlafmodusinformationen für zwei oder mehr Nebeneinheiten "auf einmal" signalisiert werden können, und daß insbesondere jede Nebeneinheit durch die Schlafmodusinformationen in dem Steuerrahmen eines aktuellen Multirahmens für eine Zeitdauer in den Schlafmodus versetzt werden können, die vorzugsweise länger als ein Multirahmen ist. Selbst wenn jedoch eine Zeitdauer gleich einem Multirahmen als Schlafzeit in dem Steuerrahmen des aktuellen Multirahmens verfügt wird, kann eine Nebeneinheit unmittelbar nach Empfang des Steuerrahmens ihre Aktivitäten unmittelbar bis zum Beginn des nächsten Multirahmens, an dem der Steuerrahmen desselben steht, beenden, um möglichst viel Strom zu sparen. Eine Nebeneinheit muß daher nicht z. B. einen ganzen Multirahmen lang empfangsbereit sein, um festzustellen, ob ein Schlafmodus für dieselbe signalisiert wird, sondern nur bis zum Empfang und zur Decodierung des Steuerrahmens des aktuellen Multirahmens.

Bevorzugte Ausführungsbeispiele der vorliegenden Erfindung werden nachfolgend Bezug nehmend auf die beiliegenden Zeichnungen detailliert erläutert. Es zeigen:
- Fig. 1: ein Blockschaltbild einer erfindungsgemäßen Zentraleinheit;
- Fig. 2: ein Blockschaltbild einer erfindungsgemäßen Nebeneinheit;
- Fig. 3: eine schematische Darstellung eines Multirahmens mit einem Steuerrahmen (SR), mehreren Nutzrahmen (DL1, UL1, DL2, DL2, UL2) und einem Freirahmen (FR); und
- Fig. 4: eine schematische Darstellung einer Folge von Multirahmen, wobei Nebeneinheiten unterschiedlich lange in einen Schlafmodus geschickt werden.

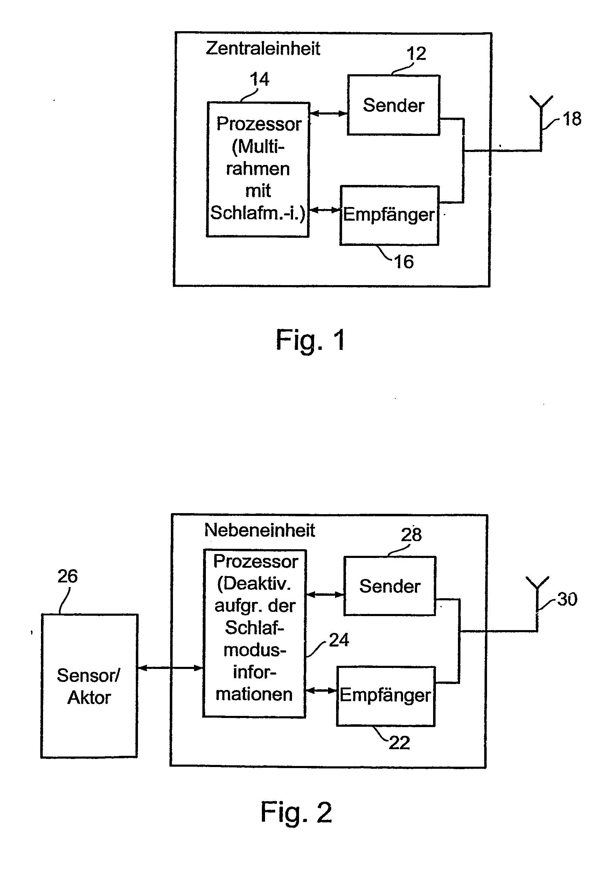

Eine erfindungsgemäße Zentraleinheit, wie sie in Fig. 1 gezeigt ist, umfaßt einen Sender 12 zum Senden einer Folge von Multirahmen, einen Prozessor 14 zum Erzeugen der Folge von Multirahmen und vorzugsweise, wenn eine bidirektionale Kommunikation vorhanden ist, einen Empfänger 16 zum Empfangen von Daten von Nebeneinheiten, wobei insbesondere der Fall einer drahtlosen Funkstrecke zwischen der Zentraleinheit und einer Nebeneinheit betrachtet wird, wie es durch eine Antenne 18 in Fig. 1 symbolisiert ist. Es sei jedoch darauf hingewiesen, daß die vorliegende Erfindung auch auf leitungsgebundene Kommunikationssysteme angewendet werden kann.

Der Prozessor 14 ist ausgebildet, um eine Folge von Multirahmen zu erzeugen und dem Sender 12 zu übermitteln, wobei ein Multirahmen der Folge von Multirahmen einen Steuerrahmen (SR) und zumindest einen Nutzrahmen (NR) aufweist, wobei der Steuerrahmen Schlafmodusinformationen für die eine oder die mehreren Nebeneinheiten aufweist, wobei die Schlafmodusinformationen auf eine Zeitdauer hinweisen, in der die zumindest eine Nebeneinheit in einem Schlafmodus sein soll. Die Zeitdauer ist mindestens so groß, wie die Zeitdauer der Nutzrahmen in dem aktuell betrachteten Multirahmen oder vorzugsweise das 2, 3, ..., n-fache eines Multirahmens. Damit kann auf einfache Weise signalisiert werden, wie viele Multirahmen eine Nebeneinheit im Schlafmodus sein soll.

Der Prozessor 14 der erfindungsgemäßen Zentraleinheit von Fig. 1 ist ferner ausgebildet, um in dem Fall, in dem der Zentraleinheit mehrere Nebeneinheiten zugeordnet sind, Steuermodusinformationen für die unterschiedlichen Nebeneinheiten zu umfassen, wobei Steuermodusinformationen beispielsweise die Datenrate, die Verzögerung, einen Fehlerschutz, eine Verschlüsselung etc. für bestimmte Nebeneinheiten individuell angeben sollen.

Der Steuerrahmen umfaßt ferner, wenn dies erwünscht ist, Schlafmodusinformationen für mehrere der Zentraleinheit zugeordnete Nebeneinheiten, die in dem aktuellen Multirahmen benötigt werden, derart, daß Schlafmodusinformationen ausschließlich im Steuerrahmen und nicht in der - für eine große Anzahl von Nebeneinheiten - beträchtlichen Anzahl von Nutzrahmen enthalten sind. Damit wissen sämtliche Nebeneinheiten immer genau, daß Schlafmodusinformationen nur im Steuerrahmen auftreten können. Dies hat zwei Vorteile. Der erste Vorteil besteht darin, daß Nebeneinheiten, denen ein Schlafmodus signalisiert worden ist, sofort nach Empfang des Steuerrahmens in den Schlafmodus gehen können und maximal viel Strom sparen können, oder daß Nebeneinheiten, denen keine Schlafmodusinformation zugewiesen worden ist, genau wissen, wann ihnen ein Nutzrahmen zum Empfang oder zum Senden zugeordnet ist, derart, daß diese noch in der Zeitdauer, die sie auf ihren Nutzrahmen warten müssen, ebenfalls in einen Schlafmodus gehen können oder, falls die Zeit hierfür zu kurz ist, doch zumindest ihre Aktivität herunterfahren können, um Strom zu sparen.

Es sei darauf hingewiesen, daß in einem Steuer-Rahmen nicht nur Schlafinformationen für eine Nebeneinheit, daß dieselbe z. B. in dem nächsten Multirahmen schlafen soll, sondern auch Steuer-Informationen für den aktuellen Multirahmen umfassen können. So kann ein Steuerrahmen der Nebeneinheit z. B. signalisieren, daß sie im zweiten Nutzrahmen einen Uplink-Rahmen hat, d. h. daß sie Daten zur Zentraleinheit senden darf, und daß sie im fünften Nutzrahmen einen Downlink-Rahmen hat, in dem sie Daten von der Zentraleinheit zu erwarten hat. Der Steuerrahmen kann ferner signalisieren, daß die Nebeneinheit im nächsten Multirahmen schlafen soll.

Der Prozessor 14 von Fig. 1 ist ferner ausgebildet, um unterschiedliche Nutzrahmen für unterschiedliche Kommunikationsrichtungen zuzuweisen, derart, daß - abgesehen von dem Freirahmen - eine sicher kollisionsfreie Kommunikation mit minimalem Signalisierungsaufwand erreicht ist.

Die Zuweisung unterschiedlicher Anzahlen von Nutzrahmen zu verschiedenen Nebeneinheiten - zusätzlich zu der Möglichkeit des selektiven Signalisierens eines Schlafmodus in einem Multirahmen - liefert eine Möglichkeit dahingehend, den unterschiedlichsten Datenratenanforderungen gerecht zu werden, die sich insbesondere bei einem körpernahen Netz ergeben. Während eine möglichst durchgehende Übertragung von EKG-Daten von einer EKG-Nebeneinheit zur Zentraleinheit wünschenswert sein dürfte, ist eine derart häufige Übertragung der Körpertemperatur von einer Temperatur-Nebeneinheit zur Zentraleinheit nicht nötig. Wird die Temperatur nur ganz selten benötigt, so kann die Temperatur-Nebeneinheit durch den Schlafmodus immer eine bestimmte Anzahl von Multirahmen in einen Schlafzustand versetzt werden, um keine Leistung zu verbrauchen. Damit wird sichergestellt, daß eine solche Nebeneinheit auch mit einer weniger leistungskräftigen Batterie beispielsweise versorgt werden kann, oder sogar batterielos betrieben werden kann, wenn sie so ausgestaltet ist, daß sie ihre Sendeleistung aus einem externen Funkfeld entziehen kann.

Selbst wenn die Temperatur-Nebeneinheit nicht im Schlafmodus ist, wird sie dennoch zur Übertragung eines Temperaturwerts wenig Übertragungskapazität benötigen. Dieser Situation kann man dadurch gerecht werden, daß der Temperatur-Nebeneinheit lediglich ein Nutzrahmen zur Übertragung zur Sendeeinheit zugewiesen wird, während beispielsweise einer EKG-Nebeneinheit mehrere Nutzrahmen in einem Multirahmen zugewiesen werden.

Dasselbe trifft für eine Kommunikation von der Zentraleinheit zur Nebeneinheit zu. Typischerweise wird in dem Fall, in dem die Nebeneinheiten Sensoren umfassen, ein Kommunikationsbedarf von der Zentraleinheit zur Nebeneinheit relativ gering sein, beispielsweise um bestimmte Parameter der Nebeneinheit einzustellen, wie z. B. Meßgenauigkeit, Meßhäufigkeit etc. Dieser Situation kann man wiederum dadurch gerecht werden, daß die Länge eines Nutzrahmens relativ klein gewählt wird, und daß zur Datenübertragung von einer Nebeneinheit zu der Zentraleinheit viele Nutzrahmen zugewiesen werden, während zu einer Datenübertragung von der Zentraleinheit zur Nebeneinheit nur ein einziger Nutzrahmen zugewiesen wird.

Fig. 2 zeigt eine Nebeneinheit, die zur Kommunikation mit einer wie in Fig. 1 dargestellten Zentraleinheit geeignet ist. Die Nebeneinheit umfaßt einen Empfänger 22, um einen Steuerrahmen eines Multirahmens zu empfangen. Die Nebeneinheit umfaßt ferner einen Prozessor 24 zum Erhalten des von dem Empfänger 22 empfangenen Steuerrahmens und zum Extrahieren der für die Nebeneinheit bestimmten Schlafmodusinformationen in dem Steuerrahmen. Der Prozessor 24 ist ferner vorgesehen, um den Empfänger in der Zeitdauer, die durch die Schlafmodusinformationen gegeben ist, zu deaktivieren, um einen Energieverbrauch der Nebeneinheit gegenüber einem Betrieb mit aktiviertem Empfänger zu reduzieren. In der Nebeneinheit direkt enthalten oder, wie es in Fig. 2 gezeigt ist, als externe Einrichtung kann der Nebeneinheit ein Sensor und/oder Aktor 26 zugeordnet sein, dessen Daten in den Prozessor 24 einspeisbar sind, um mittels eines Senders 28 von der Nebeneinheit von Fig. 2 zu der Zentraleinheit von Fig. 1 z. B. mittels einer Antenne 30 übertragen zu werden.

Wenn die Nebeneinheit einen Steuerrahmen eines Multirahmens empfängt, so kann der Prozessor 24 die Schlafmodusinformationen in dem Steuerrahmen untersuchen, um festzustellen, ob für die aktuelle Nebeneinheit Schlafmodusinformationen in dem Steuerrahmen enthalten sind. Ist dies der Fall, so wird der Prozessor 24 den Empfänger 22 abschalten, da ohnehin in der durch die Schlafmodusinformationen definierten Zeitdauer, die vorzugsweise eine Mehrzahl von Multirahmen umfaßt, ohnehin keine Informationen mehr zu der gerade betrachteten Nebeneinheit übertragen werden. Der Prozessor 24 wird ferner den Sender 28 ebenfalls ausschalten, da in der Zeit, in der die Nebeneinheit im Schlafmodus sein soll, auch keine Daten von der Zentraleinheit erwartet werden, also auch keine Daten abgesendet werden müssen, falls ein normaler Betrieb vorliegt. Der Prozessor 24 kann sich selber ebenfalls in einen Sparmodus versetzen, in dem er lediglich eine Zeitüberwachung durchführen muß, um sich dann wieder in einen aktiven Zustand zu versetzen, wenn die Schlafmodus-Zeitdauer verstrichen ist. Je nach Ausführungsbeispiel kann der Prozessor 24 in dem Fall, in dem die Nebeneinheit in einen Schlafmodus versetzt werden soll, den Sensor 26 vollständig deaktivieren. Die Nebeneinheit wird daher insgesamt in einen Energiesparmodus gebracht, der darin besteht, daß der Prozessor noch eine Zeitmeßfunktion hat, um festzustellen, wann die Schlafmodus-Zeitdauer wieder vorbei ist.

Bei einem bevorzugten Ausführungsbeispiel der vorliegenden Erfindung enthält jedoch der Multirahmen, der von der Zentraleinheit abgesendet wird, einen Frei-Rahmen oder auch Random-Rahmen. Dieser Rahmen ist dafür vorgesehen, daß auch eine Nebeneinheit die im Schlafmodus ist, Notfallinformationen zu der Zentraleinheit übermitteln kann. Hierzu wird es bevorzugt, daß der Prozessor 24 den Sensor 26 nicht vollständig abschaltet, sondern in einen Notfallüberwachungsmodus bringt, der gleich dem normalen Betrieb des Sensors/Aktors 26 sein kann. Dies bedeutet, daß der Sensor/Aktor 26 unabhängig davon, ob die Nebeneinheit im Schlafmodus ist oder nicht, dauernd Daten erfaßt oder a-giert. Darüber hinaus ist der Prozessor 24 ebenfalls in einem solchen Aktivitätszustand, daß er zwar seine Schnittstelle mit dem Sender und dem Empfänger nicht aktiv hält, daß er jedoch seine Schnittstelle zum Sensor/Aktor 26 aktiv hält, um die Ausgangsdaten des Sensors/Aktors dauernd zu überwachen, um z. B. anhand vorgegebener Notfallschwellen zu erkennen, ob ein Notfall vorliegt oder nicht. Erkennt der Prozessor 24 einen Notfall, beispielsweise eine starke Veränderung im EKG eines Patienten oder einen starken Temperaturanstieg innerhalb einer bestimmten Zeit, so wird der Prozessor 24 den Sender 28 aktivieren, um in einem Freirahmen Daten zur Zentraleinheit zu übermitteln, die auf den Notfall hinweisen. Dieses Prozedere ist dann ausreichend, wenn die Multirahmenlänge konstant ist, und wenn die Zeitmeßeinrichtung der Nebeneinheit ausreichend genau ist, um den Freirahmen selbsttätig berechnen zu können. Ist dies nicht der Fall, weil beispielsweise das Zeitnormal in der Nebeneinheit nicht besonders hochwertig ist, oder wenn eine nicht-konstante Multirahmenlänge verwendet wird, so wird der Prozessor 24 in einer Notfallsituation auch den Empfänger 22 aktivieren, damit er einen Steuerrahmen empfängt und ausgehend von dem Steuerrahmen gegebenenfalls unter Verwendung der Länge des aktuellen Multirahmens den Beginn eines Freirahmens ermittelt, um dann in diesem Freirahmen, Notfallmeldungen zur Zentraleinheit absetzen zu können.

Zur Erfassung von Notfällen ist oftmals nicht die gesamte Datenmenge erforderlich, die in einem Normalbetrieb von einem Sensor und/oder Aktor erfaßt wird. Daher kann der Prozessor 24 auch ausgebildet sein, um den Sensor/Aktor in einem Notfall-Betriebsmodus zu betreiben, wenn die Nebeneinheit im Schlafmodus ist. In einem solchen Notfall-Betriebsmodus kann der Sensor z. B. angeordnet sein, um eine Temperatur nur jede Minute statt jeder Sekunde zu erfassen etc. Sämtliche Notfall-Modi sind dafür vorgesehen, um Strom zu sparen, damit Batterien in der Nebeneinheit so klein als möglich gehalten werden können, oder daß auf Batterien ganz verzichtet werden kann, wenn die Leistung aus den Funkfeld z. B. der Zentraleinheit ausreichend ist, um einen sicheren Betrieb der Nebeneinheit zu garantieren.

Insbesondere bei Anwendungen der vorliegenden Erfindung für körpernahe Funknetzwerke ist es wesentlich, daß die Sensoren und damit deren Batterien bzw. Leistungsversorgung an sich möglichst klein gehalten ist, da diese Sensoren auch Sensoren sein können, die einer Person eingepflanzt sind, wie z. B. ein Herzschrittmacher oder eine Hörgerätesteuerung, die zwar nicht eingepflanzt ist, jedoch sehr nah am Körper getragen werden muß.

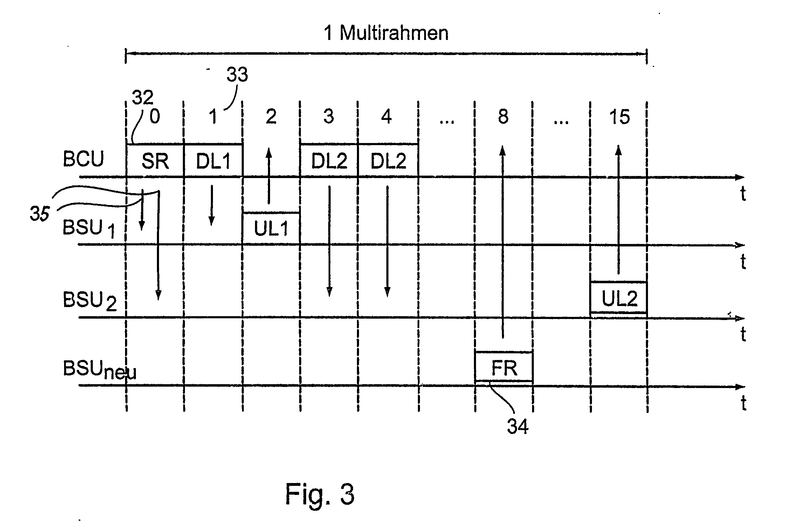

Fig. 3 zeigt ein Zeitdiagramm zum Darstellen eines Multirahmens. In dem in Fig. 3 skizzierten System existiert eine Zentraleinheit, die auch als BCU (BCU = Body Control Unit) bezeichnet wird. Darüber hinaus existieren eine erste Nebeneinheit BSU₁ und eine zweite Nebeneinheit BSU₂, wobei "BSU" für Body Sensor Unit steht. Darüber hinaus ist in Fig. 3 ein Szenario dargestellt, in dem üblicherweise zwei Nebeneinheiten BSU₁, BSU₂ bei der Zentraleinheit (BCU) registriert sind, und bei dem sich eine weitere Nebeneinheit (BSUₙₑᵤ) in dem körpernahen Funknetzwerk anmelden möchte. Der in Fig. 3 gezeigte Multirahmen umfaßt einen Steuerrahmen 32 sowie 15 Nutzrahmen, wobei ein Nutzrahmen mit der Nummer "1" mit dem Bezugszeichen 33 bezeichnet ist. In dem Steuerrahmen 32 sind sowohl Informationen für die Nebeneinheit BSU₁ als auch Informationen für die Nebeneinheit BSU₂ enthalten, wie es durch Pfeile 35 dargestellt ist. Diese Informationen sind Schlafmodusinformationen, und bei dem in Fig. 3 gezeigten Fall, bei dem beide Einheiten in dem Multirahmen aktiv sind, auch Informationen zur Zuweisung der Nutzrahmen. Ein Nutzrahmen ist entweder ein Downlink-Rahmen (DL) oder ein Uplink-Rahmen (UL), abgesehen von einem Freirahmen (FR) 34, der dafür vorgesehen ist, daß sich z. B. eine neue Nebeneinheit (BSUₙₑᵤ) bei der Zentraleinheit anmelden kann. Bei dem hier gezeigten Ausführungsbeispiel ist der Multirahmen so gestaltet, daß immer ein vorbestimmter, z. B. der achte, Nutzrahmen in der Sequenz von Rahmen in einem Multirahmen ein Freirahmen ist, während der nullte Nutzrahmen der Steuerrahmen ist, und die anderen Nutzrahmen 1 bis 7 und 9 bis 15 ebenfalls Nutzrahmen sind, die für einen Uplink-Betrieb oder einen Downlink-Betrieb geeignet sind.

Der Steuerrahmen 32 des in Fig. 3 betrachteten Multirahmens ist derart ausgestaltet, daß er der Nebeneinheit BSU₁ mitteilt, daß sie im Nutzrahmen Nr. 1 Daten von der Zentraleinheit erhält und im Nutzrahmen Nr. 2 Daten zu der Zentraleinheit senden darf. In dem Steuerrahmen 32 wird der Nebeneinheit BSU₂ signalisiert, daß sie in den Nutzrahmen 3 und 4 Daten von der Zentraleinheit erhalten muß, und daß sie im Nutzrahmen 15 Daten an die Zentraleinheit senden darf. Die beiden Nebeneinheiten BSU₁ und BSU₂ wissen nach dem Lesen der Steuerinformationen genau, daß außerhalb der Nutzrahmen, die in dem Steuerrahmen definiert sind, keine Daten gesendet werden müssen oder empfangen werden müssen, es sei denn, daß ein Notfall vorliegt. Dies bedeutet, daß sich die Nebeneinheit BSU₁ für die Nutzrahmen 3 bis 15 ohne weiteres deaktivieren kann, um Strom zu sparen. Darüber hinaus kann sich die Nebeneinheit BSU₂ für die Nutzrahmen 1, 2, 5 bis 14 ebenfalls deaktivieren, um Strom zu sparen.

Insbesondere im medizinischen Bereich besteht oft der Wunsch, weitere Sensoren hinzuzufügen. Dies muß möglich sein, ohne daß die Zentraleinheit heruntergefahren werden muß bzw. überhaupt in ihrem Betrieb gestört werden darf, da die bereits vorhandenen Nebeneinheiten möglicherweise wichtige Daten erfassen. Um dies zu ermöglichen, kann ebenfalls der Freirahmen 34 verwendet werden, der ansonsten auch für Notfallmeldungen der bereits angemeldeten Nebeneinheiten zur Verfügung steht. Um sich bei der Zentraleinheit anzumelden, muß beim erfindungsgemäßen System die weitere Nebeneinheit BSUₙₑᵤ eine Aufsynchronisation auf die Multirahmenübertragung liefern, um z. B. den Steuerrahmen 32 zu ermitteln. Typischerweise wird die zusätzlich hinzugefügte Nebeneinheit voreingestellt sein, um zu wissen, daß immer der achte Nutzrahmen in einem Multirahmen der Freirahmen ist, in dem die Zentraleinheit auf einen Uplink-Betrieb eingestellt ist. Wenn die zusätzliche Nebeneinheit BSUₙₑᵤ also den Steuerrahmen 32 erfaßt hat, so wartet sie noch sieben Nutzrahmen, um dann im achten Nutzrahmen, dem Freirahmen 34, ihr Anmeldungsprotokoll zur Zentraleinheit zu übermitteln.

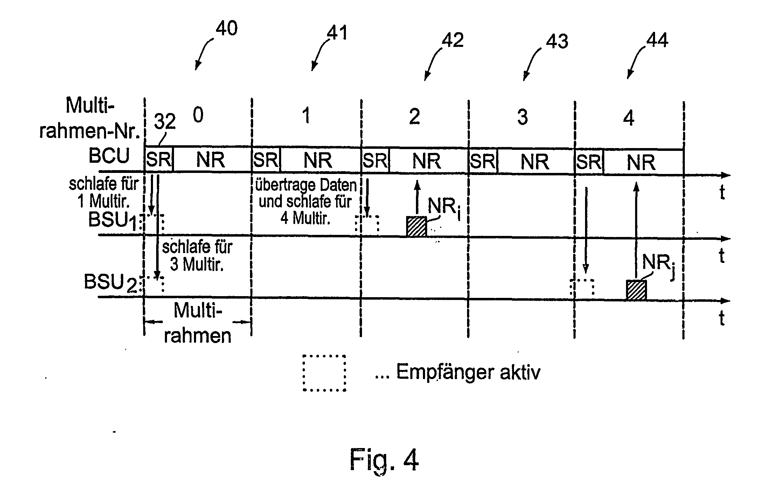

In Fig. 4 ist eine Folge von Multirahmen 40 bis 44 dargestellt. Jeder Multirahmen umfaßt einen Steuerrahmen (SR) 32 sowie eine Mehrzahl von Nutzrahmen (NR). Darüber hinaus ist in Fig. 4 der Fall gezeigt, bei dem lediglich zwei Nebeneinheiten angemeldet sind, obgleich die Anzahl der Nebeneinheiten, die bei einer Zentraleinheit registriert sind, prinzipiell beliebig ist, so lange die Prozessor- und Speicherkapazität der Zentraleinheit hierzu ausreichend ist. In dem Steuerrahmen 32 des 0-ten Multirahmens 40 von Fig. 4 sind Schlafmodusinformationen sowohl für die erste Nebeneinheit BSU₁ als auch für die zweite Nebeneinheit BSU₂ enthalten. Wie es in Fig. 4 beispielhaft dargestellt ist, bedeuten die Schlafmodusinformationen für die erste Nebeneinheit, daß diese einen Multirahmen lang schlafen soll, und zwar den darauffolgenden Multirahmen Nr. 1, der in Fig. 4 auch mit 41 bezeichnet ist. Die erste Nebeneinheit BSU₁ hat somit im Multirahmen 41 keinen aktiven Empfänger, der allgemein durch einen gestrichelten Kasten dargestellt ist.

In den Schlafmodusinformationen für die zweite Nebeneinheit BSU₂ in dem Steuerrahmen 32 des Multirahmens 40 ist ausgeführt, daß diese für drei Multirahmen schlafen soll. Die zweite Nebeneinheit BSU₂ ist daher in den Multirahmen 41, 42 und 43 im Schlafmodus.

Nachdem für die erste Nebeneinheit BSU₁ die Zeitdauer für den Schlafmodusbetrieb am Ende des ersten Multirahmens 41 abgelaufen ist, wird in dem zweiten Multirahmen 42 der Empfänger der ersten Nebeneinheit wieder aktiv, um den Steuerrahmen SR des Multirahmens 42 zu decodieren, in dem steht, daß die erste Nebeneinheit in dem i-ten Nutzrahmen NRᵢ Daten zur Zentraleinheit BCU senden soll. Darüber hinaus enthalten die Schlafmodusinformationen den Hinweis, daß die erste Nebeneinheit dann für vier Multirahmen wieder schlafen soll, weshalb in dem Multirahmen 43 und in dem Multirahmen 44, die in Fig. 4 noch gezeigt sind, der Empfänger der ersten Nebeneinheit BSU₁ nicht aktiv ist.

Dagegen aktiviert sich die zweite Nebeneinheit BSU₂ nach dem Ablauf der drei Multirahmen 41, 42 und 43 wieder im Multirahmen 44, um den Steuerrahmen SR in dem Multirahmen 44 zu lesen. Bei dem hier gezeigten Beispiel umfaßt der Steuerrahmen des Multirahmens 44 den Hinweis, daß die zweite Nebeneinheit BSU₂ im Nutzrahmen NRⱼ Daten zur Zentraleinheit BCU übertragen soll. Der Steuerrahmen des Multirahmens 44 kann dann gegebenenfalls wieder Schlafmodusinformationen für die zweite Nebeneinheit umfassen.

Das erfindungsgemäße System ist besonders geeignet für ein körpernahes Funknetzwerk, bei dem dezentrale Sensoren zur Erfassung von Vitalparametern, wie z. B. Temperatur, Blutdruck, EKG-Ableitung, ..., mit einer Zentralstation drahtlos kommunizieren.

Die Erfindung ist dahingehend vorteilhaft, daß die Zentraleinheit für das erfindungsgemäße Netz die Übertragungskapazität den einzelnen Aktor/Sensoreinheiten beliebig zuweisen kann, indem der Steuerrahmen entsprechend geschrieben wird.

Darüber hinaus wird gemäß der vorliegenden Erfindung eine periodische oder nicht-periodische Rahmenstruktur mit Multirahmen definiert. Ein Multirahmen umfaßt mehrere Rahmen, wobei ein Rahmen des Multirahmens als Steuerrahmen verwendet wird, wobei der Steuerrahmen der Zentraleinheit fest als Downlink zugewiesen ist.

In dem Steuerrahmen werden ferner die übrigen Rahmen des Multirahmens den Nebeneinheiten zugeteilt. Für jeden Rahmen wird für eine angesprochene Aktor/Sensoreinheit die Senderichtung (UL, DL) sowie die Paketgröße festgelegt.

Darüber hinaus wird allen Aktor/Sensoreinheiten in dem Kontrollrahmen von der Zentraleinheit vorgegeben, welche Aktor/Sensoreinheit wie lange, beispielsweise als Anzahl von Multirahmen, in einen Stromsparmodus gehen darf.

Die Aktivität der Aktor/Sensoreinheiten, d. h. der Nebeneinheiten, kann somit durch den Steuerrahmen genau gesteuert werden.

Der Freirahmen 34 (Fig. 3) wird für nicht geplante Zugriffe der Aktor/Sensoreinheiten freigehalten. Dort haben neue Aktor/Sensoreinheiten die Möglichkeit, sich im Funknetzwerk anzumelden. Weiterhin haben Aktor/Sensoreinheiten die Möglichkeit, trotz zugewiesener Inaktivität, also wenn sie im Schlafmodus sind, Notfallmeldungen abzusetzen. Der Zugriff auf diesen Rahmen erfolgt nach einem Zufallsprinzip oder kann über weitere Mechanismen zur Kollisionsvermeidung geregelt werden.

Der Steuerrahmen kann für eine eigene Kennung, wie z. B. ein Synchronisationswort, verfügen. Dadurch können neue Aktor/Sensoreinheiten oder Einheiten, die die Synchronisation verloren haben, einfach den Steuerrahmen detektieren und von den anderen Nutzrahmen in dem Multirahmen, und insbesondere von den Downlink-Rahmen in dem Multirahmen unterscheiden.

Die vorliegende Erfindung ist dahingehend vorteilhaft, daß die Übertragungsqualität (Datenrate für Uplink/Downlink, Verzögerung, Fehlerschutz) von der Zentraleinheit individuell für jede Aktor/Sensoreinheit gesteuert werden kann. Die zur Verfügung stehende Übertragungskapazität kann flexibel verteilt werden, also als symmetrischer oder als asymmetrischer Up-/Downlink.

Ein weiterer Vorteil der vorliegenden Erfindung besteht darin, daß die Aktor/Sensoreinheiten maximal lange inaktiv sein können, d. h. in einem Stromzustand mit minimalem Stromverbrauch, da der Sende/Empfangs-Betrieb deterministisch unter Verwendung des Multirahmen-Formats und des Steuerrahmens in dem Multirahmen gesteuert ist. Multirahmen, in denen eine Nebeneinheit im Schlafmodus ist, brauchen weder empfangen noch interpretiert werden.

Ein weiterer Vorteil der vorliegenden Erfindung besteht darin, daß alle Aktor/Sensoreinheiten gemeinsam in einem Steuerrahmen pro Multirahmen gesteuert werden. Dadurch ist ein minimaler Aufwand für die Netzwerkaktivitätssteuerung notwendig, und die Netzwerkaktivitätssteuerung ist transparent für Bedien- und Wartungspersonen.

Darüber hinaus besteht ein wesentlicher Vorteil der vorliegenden Erfindung darin, daß aufgrund des Freirahmens 34 bzw., wenn ein Multirahmen mehr Nutzrahmen bekommt, noch gegebenenfalls weitere Freirahmen pro Multirahmen für Notfallmeldungen bzw. Netzkonfigurationsänderungen z. B. durch Hinzufügen von weiteren Nebeneinheiten möglich sein können.

Obgleich im vorhergehenden periodische Multirahmenlängen beschrieben worden sind, können auch nicht-periodische Multirahmen eingesetzt werden. Darüber hinaus kann das erfindungsgemäße Konzept auch bei nicht-medizinischen Aufgaben eingesetzt werden, wobei es besonders gut für Aufgaben geeignet ist, bei denen stark unterschiedliche Datenraten verarbeitet werden müssen, und bei denen ein minimaler Stromverbrauch der Sensor/Aktoreinheiten bzw. allgemein gesagt der Nebeneinheiten von hoher Bedeutung ist.

## Patentansprüche

1. Zentraleinheit zum Kommunizieren mit einer Mehrzahl von Nebeneinheiten, mit folgenden Merkmalen:
einem Sender (12) zum Senden einer Folge von Multirahmen; und
einem Prozessor (14) zum Erzeugen der Folge von Multirahmen, wobei die Multirahmen der Folge von Multirahmen jeweils einen Steuerrahmen (32) und eine Mehrzahl von Nutzrahmen (33) aufweisen, wobei der Steuerrahmen eines aktuellen Multirahmens (40) Informationen über eine Zuweisung der Mehrzahl von Nutzrahmen zu der Mehrzahl von Nebeneinheiten und Schlafmodusinformationen für die Mehrzahl von Nebeneinheiten aufweist, wobei die Schlafmodusinformationen auf eine Zeitdauer hinweisen, in der die Nebeneinheiten (BSU1) jeweils in einem Schlafmodus sein sollen, wobei die Zeitdauer zumindest einen Multirahmen (41) umfasst, der auf den aktuellen Multirahmen (40) folgt, wobei die Schlafmodusinformationen ausschließlich in dem Steuerrahmen und nicht in der Mehrzahl von Nutzrahmen enthalten sind,
wobei die Zentraleinheit ausgebildet ist, um der zumindest einen Nebeneinheit durch die Schlafmodusinformationen in dem Steuerrahmen vorzugeben, wie lange dieselbe in den Schlafmodus gehen darf.

2. Zentraleinheit nach Anspruch 1, die zur Kommunikation mit der einen und einer weiteren Nebeneinheit vorgesehen ist,
bei der der Steuerrahmen erste Schlafmodusinformationen für die eine Nebeneinheit und zweite Schlafmodusinformationen für die weitere Nebeneinheit aufweist.

3. Zentraleinheit nach Anspruch 1 oder 2,
bei der ein Multirahmen für jede Nebeneinheit einen Downlink-Nutzrahmen für eine Kommunikation von der Zentraleinheit zu der Nebeneinheit und einen Uplink-Nutzrahmen für eine Kommunikation von der Nebeneinheit zu der Zentraleinheit aufweist,
wobei der Steuerrahmen (32) für jede Nebeneinheit Informationen darüber umfaßt, welcher Nutzrahmen in dem Multirahmen ein Uplink-Rahmen oder ein Downlink-Rahmen für jede Nebeneinheit ist.

4. Zentraleinheit nach einem der vorhergehenden Ansprüche,
bei der der Steuerrahmen für eine Kommunikation von der Nebeneinheit zu der Zentraleinheit eine Anzahl von Nutzrahmen in dem Multirahmen, die der Nebeneinheit zugewiesen ist bzw. sind, aufweist.

5. Zentraleinheit nach einem der vorhergehenden Ansprüche,
bei der der Multirahmen ferner zumindest einen Freirahmen (34) aufweist, in dem ein Empfänger der Zentraleinheit aktiv ist, wobei der Freirahmen (34) keiner speziellen Nebeneinheit zugeordnet ist, so daß die Zentraleinheit für einen Empfang einer Anmeldung einer noch nicht registrierten weiteren Nebeneinheit oder eines Notrufs einer in den Schlafmodus versetzten Nebeneinheit in dem Freirahmen vorbereitet ist.

6. Zentraleinheit nach einem der vorhergehenden Ansprüche, die für ein drahtloses Senden ausgebildet ist.

7. Zentraleinheit nach einem der vorhergehenden Ansprüche, die für ein körpernahes Netzwerk vorgesehen ist,
wobei die zumindest eine Nebeneinheit einen Sensor zur Erfassung von Vitalparametern oder einen Aktor zum direkten oder indirekten Beeinflussen eines Vitalparameters aufweist.

8. Zentraleinheit nach einem der Ansprüche 1 bis 7,
bei der in dem Steuerrahmen eine Datenmenge definiert ist, die in einem Nutzrahmen des Multirahmens zu übertragen ist.

9. Zentraleinheit nach einem der vorhergehenden Ansprüche,
bei der der Steuerrahmen eine Steuerrahmenidentifikation aufweist, die der zumindest einen Nebeneinheit bekannt ist.

10. Zentraleinheit nach einem der vorhergehenden Ansprüche,
bei der sowohl der Steuerrahmen als auch jeder Nutzrahmen in dem Multirahmen jeweils eine vorbestimmte zeitliche Länge aufweisen.

11. Zentraleinheit nach Anspruch 10,
bei der die vorbestimmte zeitliche Länge sowohl des Steuerrahmens als auch jedes Nutzrahmens gleich ist.

12. Zentraleinheit nach einem der Ansprüche 1 bis 11,
bei der der Steuerrahmen Einstellungsinformationen zum Einstellen von Parametern eines Sensors und/oder Aktors in der Nebeneinheit aufweist.

13. Zentraleinheit nach einem der vorhergehenden Ansprüche, die ferner einen Empfänger (16) aufweist, der zum Zeitpunkt des Sendens des Multirahmens nicht aktiv ist.

14. Zentraleinheit nach einem der Ansprüche 1 bis 13,
bei der die Zeitdauer größer als die Zeitdauer eines Multirahmens ist.

15. Zentraleinheit nach einem der Ansprüche 1 bis 14,
bei der die Schlafmodusinformationen eine Anzahl von Multirahmen definieren.

16. Zentraleinheit nach einem der Ansprüche 1 bis 15,
bei der der Steuer-Rahmen des Multirahmens derart ausgebildet ist, daß in dem Multirahmen, in dem eine Nebeneinheit im Schlafmodus ist, der Steuerrahmen für diese Nebeneinheit keinen Nutzrahmen signalisiert.

17. Nebeneinheit zum Kommunizieren mit einer Zentraleinheit über eine Folge von Multirahmen, wobei ein Multirahmen einen Steuerrahmen und Nutzrahmen aufweist, mit folgenden Merkmalen:
einem Empfänger (22) zum Empfangen eines aktuellen Multirahmens (40) der Folge von Multirahmen, wobei der aktuelle Multirahmen (40) einen Steuerrahmen und eine Mehrzahl von Nutzrahmen aufweist, wobei der Steuerrahmen Informationen über eine Zuweisung der Mehrzahl von Nutzrahmen zu der Mehrzahl von Nebeneinheiten und Schlafmodusinformationen für eine oder mehrere Nebeneinheiten aufweist, wobei die Schlafmodusinformationen von der Zentraleinheit vorgegeben sind und auf eine Zeitdauer hinweisen, in der die mehreren Nebeneinheiten in einem Schlafmodus sein sollen, wobei die Zeitdauer zumindest einen Multirahmen (41) umfasst, der auf den aktuellen Multirahmen (40) folgt, und wobei der Empfänger (22) lediglich betreibbar ist, wenn die Nebeneinheit nicht in dem Schlafmodus ist, wobei die Schlafmodusinformationen ausschließlich in dem Steuerrahmen und nicht in der Mehrzahl von Nutzrahmen enthalten sind; und
einem Prozessor (24) zum Interpretieren von für die Nebeneinheit in dem Steuerrahmen des aktuellen Multirahmens (40) enthaltenen Schlafmodusinformationen und zum Deaktivieren des Empfängers (22) in der Zeitdauer, die zumindest den dem aktuellen Multirahmen (40) folgenden Multirahmen (41) umfasst, um einen Energieverbrauch der Nebeneinheit gegenüber einem Betrieb mit aktiviertem Empfänger zu reduzieren.

18. Nebeneinheit nach Anspruch 17, die ferner folgende Merkmale aufweist:
einen Sensor und/oder Aktor (26) zum Erfassen von Daten bzw. zum Ausführen einer Handlung.

19. Nebeneinheit nach Anspruch 18,
bei der der Sensor und/oder Aktor (26) in der Zeitdauer aktiv ist, um einen Notfall zu erfassen,
und bei der der Prozessor (24) ausgebildet ist, um innerhalb der Zeitdauer den Sender (28) zu aktivieren, um eine Notfallmeldung zu übertragen.

20. Nebeneinheit nach Anspruch 19,
bei der der Multirahmen einen Freirahmen (34) aufweist, und bei der der Prozessor ausgebildet ist, um im Falle eines Notfalls den Empfänger (22) zu aktivieren, um einen Freirahmen (34) in einem Multirahmen zu erfassen, und um den Sender (28) zu aktivieren, um die Notfallmeldung in dem erfaßten Freirahmen (34) zu übertragen.

21. Nebeneinheit nach einem der Ansprüche 17 bis 20,
bei der der Prozessor ausgebildet ist, um aus dem Steuerrahmen des Multirahmens einen oder mehrere Nutzrahmen zu extrahieren, in denen der Empfänger (22) zum Empfangen von Daten von der Zentraleinheit bereit ist, und in dem der Sender (28) zum Senden von Daten von der Nebeneinheit zu der Zentraleinheit bereit ist.

22. Nebeneinheit nach Anspruch 21,
bei der der Empfänger (22) und der Sender (28) in Nutzrahmen, in denen sie nicht bereit sind, deaktiviert sind.

23. Nebeneinheit nach einem der Ansprüche 18 bis 22,
bei der in der Zeitdauer der Aktor vollständig deaktiviert wird und der Sensor in einen Sparmodus gesetzt wird, wobei der Sparmodus so gestaltet ist, daß eine Notfallsituation erfaßbar ist, daß jedoch ein Stromverbrauch kleiner ist als in dem Fall, in dem Nutzdaten für einen Nutzrahmen erfaßt werden.

24. Nebeneinheit nach einem der Ansprüche 17 bis 23,
bei der die Zeitdauer eine Mehrzahl von Multirahmen umfaßt, und bei der in der Mehrzahl von Multirahmen der Empfänger (22) deaktiviert ist.

25. Verfahren zum Kommunizieren einer Zentraleinheit mit einer Mehrzahl von Nebeneinheiten, mit folgenden Schritten:
Erzeugen einer Folge von Multirahmen, wobei ein Multirahmen der Folge von Multirahmen einen Steuerrahmen (32) und eine Mehrzahl von Nutzrahmen (33) aufweist, wobei der Steuerrahmen eines aktuellen Multirahmens (40) Informationen über eine Zuweisung der Mehrzahl von Nutzrahmen zu der Mehrzahl von Nebeneinheiten und Schlafmodusinformationen für die zumindest eine Nebeneinheit (BSU1) aufweist, wobei die Schlafmodusinformationen auf eine Zeitdauer hinweisen, in der die Nebeneinheiten jeweils in einem Schlafmodus sein sollen, wobei die Zeitdauer zumindest einen Multirahmen (41) umfasst, der auf den aktuellen Multirahmen (40) folgt, wobei die Schlafmodusinformationen ausschließlich in dem Steuerrahmen und nicht in der Mehrzahl von Nutzrahmen enthalten sind; und
Senden der Folge von Multirahmen,
wobei der zumindest einen Nebeneinheit von der Zentraleinheit durch die Schlafmodusinformationen in dem Steuerrahmen vorgegeben wird, wie lange dieselbe in den Schlafmodus gehen darf.

26. Verfahren zum Betreiben einer Nebeneinheit zum Kommunizieren einer Nebeneinheit mit einer Zentraleinheit über eine Folge von Multirahmen, wobei ein Multirahmen einen Steuerrahmen und Nutzrahmen aufweist, mit folgenden Schritten:
Empfangen eines aktuellen Multirahmens (40), wobei der aktuelle Multirahmen einen Steuerrahmen und Nutzrahmen aufweist, wobei der Steuerrahmen des aktuellen Multirahmens (40) Informationen über eine Zuweisung der Mehrzahl von Nutzrahmen zu der Mehrzahl von Nebeneinheiten und Schlafmodusinformationen für eine oder mehrere Nebeneinheiten (BSU1) aufweist, wobei die Schlafmodusinformationen von der Zentraleinheit vorgegeben sind und auf eine Zeitdauer hinweisen, in der die mehreren Nebeneinheiten in einem Schlafmodus sein sollen, wobei die Zeitdauer zumindest einen Multirahmen (41) umfasst, der auf den aktuellen Multirahmen (40) folgt, wobei die Schlafmodusinformationen ausschließlich in dem Steuerrahmen und nicht in der Mehrzahl von Nutzrahmen enthalten sind;
Interpretieren von für die Nebeneinheit (BSU1) in dem Steuerrahmen enthaltenen Schlafmodusinformationen und Deaktivieren eines Empfängers (22) der Nebeneinheit (BSU1) in der Zeitdauer, die zumindest den Multirahmen (41) umfasst, der auf den aktuellen Multirahmen (40) folgt.

## Claims

1. A central unit for communicating with a plurality of subunits, comprising:
a transmitter (12) for transmitting a series of multi-frames; and
a processor (14) for generating the series of multi-frames, wherein the multi-frames of the series of multi-frames each comprise a control frame (32) and a plurality of useful frames (33), wherein the control frame of a current multi-frame (40) comprises information about an allocation of the plurality of useful frames to the plurality of subunits and sleep mode information for the plurality of subunits, wherein the sleep mode information refers to a time period during which the subunits (BSU1) are each to be in a sleep mode, wherein the time period comprises at least one multi-frame (41), which follows the current multi-frame (40), wherein the sleep mode information is exclusively included in the control frame and not in the plurality of useful frames,
wherein the central unit is implemented to indicate to the at least one subunit, by the sleep mode information in the control frame, how long the same is allowed to go into the sleep mode.

2. The central unit according to claim 1, which is provided for communication with the one and one further subunit,
wherein the control frame comprises first sleep mode information for the subunit and second sleep mode information for the further subunit.

3. The central unit according to claim 1 or 2, wherein a multi-frame comprises a downlink useful frame for communication from the central unit to the subunit for every subunit, and an uplink useful frame for communication from the subunit to the central unit,
wherein the control frame (32) comprises information for every subunit about which useful frame in the multi-frame is an uplink frame or a downlink frame for every subunit.

4. The central unit according to one of the previous claims,
wherein the control frame comprises a number of useful frames in the multi-frame that is or are allocated to the subunit, for communication from the subunit to the central unit.

5. The central unit according to one of the previous claims,
wherein the multi-frame further comprises at least one vacant frame (34), in which a receiver of the central unit is active, wherein the vacant frame (34) is not allocated to a specific subunit, so that the central unit is prepared for receiving a request of a not yet registered further subunit or an emergency call of a subunit placed into the sleep mode in the vacant frame.

6. The central unit according to one of the previous claims, which is implemented for wireless transmission.

7. The central unit according to one of the previous claims, which is provided for a body area network,
wherein the at least one subunit comprises a sensor for detecting vital parameters or an actor for direct or indirect influencing of a vital parameter.

8. The central unit according to one of claims 1 to 7,
wherein an amount of data is defined in the control frame, which is to be transferred in a useful frame of the multi-frame.

9. The central unit according to one of the previous claims,
wherein the control frame comprises a control frame identification, which is known to the at least one subunit.

10. The central unit according to one of the previous claims,
wherein both the control frame and any useful frame in the multi-frame each have a predetermined temporal length.

11. The central unit according to claim 10,
wherein the predetermined temporal length of both the control frame and every useful frame is the same.

12. The central unit according to one of claims 1 to 11,
wherein the control frame comprises adjustment information for adjusting parameters of a sensor and/or actor in the subunit.

13. The central unit according to one of the previous claims,
further comprising a receiver (16), which is not active at the time of transmitting the multi-frame.

14. The central unit according to one of claims 1 to 13,
wherein the time period is longer than the time period of a multi-frame.

15. The central unit according to one of claims 1 to 14,
wherein the sleep mode information defines a plurality of multi-frames.

16. The central unit according to one of claims 1 to 15,
wherein the control frame of the multi-frame is implemented such that in the multi-frame, in which a subunit is in the sleep mode, the control frame for this subunit signalizes no useful frame.

17. A subunit for communicating with a central unit via a series of multi-frames, wherein a multi-frame comprises a control frame and useful frames, comprising:
a receiver (22) for receiving a current multi-frame(40) of the series of multi-frames, wherein the current multi-frame(40) comprises a control frame and a plurality of useful frames, wherein the control frame comprises information about an allocation of the plurality of useful frames to the plurality of subunits and sleep mode information for one or several subunits, wherein the sleep mode information is predetermined by the central unit and refers to a time period during which the several subunits are to be in a sleep mode, wherein the time period comprises at least one multi-frame (41), which follows the current multi-frame (40), and wherein the receiver (22) is merely operable when the subunit is not in the sleep mode, wherein the sleep mode information is exclusively included in the control frame and not in the plurality of useful frames; and
a processor (24) for interpreting sleep mode information for the subunit included in the control frame of the current multi-frame (40), and for deactivating the receiver (22) during the time period which comprises at least the multi-frame (41), which follows the current multi-frame (40), to reduce energy consumption of the subunit compared to an operation with activated receiver.

18. The subunit according to claim 17, further comprising:
a sensor and/or actor (26) for detecting data or executing an action, respectively.

19. The subunit according to claim 18,
wherein the sensor and/or actor (26) is active in the time period to detect an emergency,
and wherein the processor (24) is implemented to activate the transmitter (28) within the time period to transmit an emergency message.

20. The subunit according to claim 19,
wherein the multi-frame comprises a vacant frame (34), and wherein the processor is implemented to activate the receiver (22) in the case of an emergency, to detect a vacant frame (34) in a multi-frame, and to activate the transmitter (28) for transmitting the emergency message in the detected vacant frame (34).

21. The subunit according to one of claims 17 to 20,
wherein the processor is implemented to extract one or several useful frames from the control frame of the multi-frame, in which the receiver (22) is ready for receiving data from the central unit, and in which the transmitter (28) is ready for transmitting data from the subunit to the central unit.

22. The subunit according to claim 21,
wherein the receiver (22) and the transmitter (28) are deactivated in useful frames, in which they are not ready.

23. The subunit according to one of claims 18 to 22,
wherein the actor is fully activated during the time period, and the sensor is placed into saving mode, wherein the saving mode is designed such that an emergency situation is detectable, but that the current consumption is lower than in the case where useful data are detected for a useful frame.

24. The subunit according to one of claims 17 to 23, wherein the time period comprises a plurality of multi-frames, and wherein the receiver (22) is deactivated in the plurality of multi-frames.

25. A method for communication of a central unit with a plurality of subunits, comprising the steps of:
generating a series of multi-frames, wherein the multi-frame of the series of multi-frames comprises a control frame (32) and a plurality of useful frames (33), wherein the control frame of a current multi-frame (40) comprises information about an allocation of the plurality of useful frames to the plurality of subunits and sleep mode information for the at least one subunit (BSU1), wherein the sleep mode information refers to a time period during which the subunits are each to be in a sleep mode, wherein the time period comprises at least one multi-frame (41), which follows the current multi-frame (40), wherein the sleep mode information is exclusively included in the control frame and not in the plurality of useful frames; and
transmitting the series of multi-frames,
wherein the sleep mode information in the control frame indicates to the at least one subunit of the central unit, how long the same is allowed to go into sleep mode.

26. A method for operating a subunit for communication of a subunit with a central unit via a series of multi-frames, wherein a multi-frame comprises a control frame and useful frame, comprising the steps of:
receiving a current multi-frame (40), wherein the current multi-frame comprises a control frame and useful frame, wherein the control frame of the current multi-frame (40) comprises information about an allocation of the plurality of useful frames to the plurality of subunits and sleep mode information for one or several subunits (BSU1), wherein the sleep mode information is predetermined by the central unit and refers to a time period during which the several subunits are to be in a sleep mode, wherein the time period comprises at least one multi-frame (41), which follows the current multi-frame (40), wherein the sleep mode information is exclusively included in the control frame and not in the plurality of useful frames;
interpreting sleep mode information included in the control frame for the subunit (BSU1), and deactivating a receiver (22) of the subunit (BSU1) during the time period that comprises at least the multi-frame (41), which follows the current multi-frame (40).

## Revendications

1. Unité centrale destinée à communiquer avec une pluralité d'unités secondaires, aux caractéristiques suivantes :
un émetteur (12) destiné à émettre une succession de trames multiples ; et
un processeur (14) destiné à générer la succession de trames multiples, les trames multiples de la succession de trames multiples présentant, chacune, une trame de commande (32) et une pluralité de trames utiles (33), la trame de commande d'une trame multiple actuelle (40) présentant des informations sur une association de la pluralité de trames utiles à la pluralité d'unités secondaires et des informations de mode de sommeil pour la pluralité d'unités secondaires, les informations de mode de sommeil indiquant une période pendant laquelle les unités secondaires (BSU1) doivent être, chacune, dans un mode de sommeil, la période comportant au moins une trame multiple (41) qui suit la trame multiple actuelle (40), les informations de mode de sommeil étant contenues exclusivement dans la trame de commande et non pas dans la pluralité de trames utiles,
l'unité centrale étant réalisée de manière à prédéterminer pour l'au moins une unité secondaire, par les informations de mode de sommeil dans la trame de commande, le temps pendant lequel celle-ci peut fonctionner en mode de sommeil.

2. Unité centrale selon la revendication 1, prévue pour la communication avec l'une et une autre unité secondaire,
dans laquelle la trame de commande présente des premières informations de mode de sommeil pour l'une unité secondaire et des deuxièmes informations de mode de sommeil pour l'autre unité secondaire.

3. Unité centrale selon la revendication 1 ou 2,
dans laquelle une trame multiple présente, pour chaque unité secondaire, une trame utile de liaison descendante pour une communication de l'unité centrale vers l'unité secondaire et une liaison ascendante pour une communication de l'unité secondaire vers l'unité centrale,
la trame de commande (32) comportant, pour chaque unité secondaire, des informations sur la trame utile dans la trame multiple qui est une trame de liaison ascendante ou une trame de liaison descendante pour chaque unité secondaire.

4. Unité centrale selon l'une des revendications précédentes,
dans laquelle la trame de commande présente, pour une communication de l'unité secondaire vers l'unité centrale, un nombre de trames utiles dans la trame multiple associées à l'unité secondaire.

5. Unité centrale selon l'une des revendications précédentes,
dans laquelle la trame multiple présente, par ailleurs, au moins une trame libre (34) dans laquelle est actif un récepteur de l'unité centrale, la trame libre (34) n'étant pas associée à une unité secondaire particulière, de sorte que l'unité centrale soit préparée pour une réception d'une demande de connexion d'une autre unité secondaire non encore enregistrée ou d'un appel de secours d'une unité secondaire placée en mode de sommeil dans la trame libre.

6. Unité centrale selon l'une des revendications précédentes, qui est réalisée pour une transmission sans fil.

7. Unité centrale selon l'une des revendications précédentes, qui est prévue pour un réseau à proximité du corps,
l'au moins une unité secondaire présentant un capteur destiné à détecter des paramètres vitaux ou un actionneur pour exercer une influence directe ou indirecte sur un paramètre vital.

8. Unité centrale selon l'une des revendications 1 à 7,
dans laquelle est définie, dans la trame de commande, une quantité de données qui doit être transmise dans une trame utile de la trame multiple.

9. Unité centrale selon l'une des revendications précédentes,
dans laquelle la trame de commande présente une identification de trame de commande qui est connue de l'au moins une unité secondaire.

10. Unité centrale selon l'une des revendications précédentes,
dans laquelle tant la trame de commande que chaque trame secondaire dans la trame multiple présentent, chacune, une longueur dans le temps prédéterminée.

11. Unité centrale selon la revendication 10,
dans laquelle la longueur dans le temps prédéterminée tant de la trame de commande que de chaque trame secondaire est identique.

12. Unité centrale selon l'une des revendications 1 à 11,
dans laquelle la trame de commande présente des informations de réglage destinées à régler les paramètres d'un capteur et/ou d'un actionneur dans l'unité secondaire.

13. Unité centrale selon l'une des revendications précédentes, présentant, par ailleurs, un récepteur (16) qui, au moment de la transmission de la trame multiple, n'est pas actif.

14. Unité centrale selon l'une des revendications 1 à 13,
dans laquelle la durée est supérieure à la durée d'une trame multiple.

15. Unité centrale selon l'une des revendications 1 à 14,
dans laquelle les informations de mode de sommeil définissent un nombre de trames multiples.

16. Unité centrale selon l'une des revendications 1 à 15,
dans laquelle la trame de commande de la trame multiple est réalisée de sorte que, dans la trame multiple dans laquelle une unité secondaire est en mode de sommeil, la trame de commande pour cette unité secondaire ne signale pas de trame utile.

17. Unité secondaire pour communiquer avec une unité centrale par l'intermédiaire d'une succession de trames multiples, une trame multiple présentant une trame de commande et des trames utiles, aux caractéristiques suivantes :
un récepteur (22) destiné à recevoir une trame multiple actuelle (40) de succession de trames multiples, la trame multiple actuelle (40) présentant une trame de commande et une pluralité de trames utiles, la trame de commande présentant des informations sur une association de la pluralité de trames utiles à la pluralité d'unités secondaires et des informations de mode de sommeil pour une ou plusieurs unités secondaires, les informations de mode de sommeil étant prédéterminées par l'unité centrale et indiquant une période pendant laquelle les plusieurs unités secondaires doivent être en un mode de sommeil, la période comportant au moins une trame multiple (41) qui suit la trame multiple actuelle (40), et le récepteur (22) ne pouvant fonctionner que lorsque l'unité secondaire n'est pas en mode de sommeil, les informations de mode de sommeil étant contenues exclusivement dans la trame de commande et non pas dans la pluralité de trames utiles ; et
un processeur (24) destiné à interpréter les informations de mode de sommeil pour l'unité secondaire contenues dans la trame de commande de la trame multiple actuelle (40) et à désactiver le récepteurs (22) dans la période comportant au moins la trame multiple (41) suivant la trame multiple actuelle (40), afin de réduire une consommation d'énergie de l'unité secondaire par rapport à un fonctionnement à récepteur activé.

18. Unité secondaire selon la revendication 17, présentant, par ailleurs, les caractéristiques suivantes :
un capteur et/ou actionneur (26) destiné à détecter des données ou à effectuer une manipulation.

19. Unité secondaire selon la revendication 18,
dans laquelle le capteur et/ou l'actionneur (26) est actif dans la période, pour détecter un cas de secours,
et dans lequel le processeur (24) est réalisé de manière à activer l'émetteur (28) dans la période, pour transmettre un message de secours.

20. Unité secondaire selon la revendication 19,
dans laquelle la trame multiple présente une trame libre (34), et dans lequel le processeur est réalisé de manière à activer, en cas de secours, le récepteur (22) pour détecter une trame libre (34) dans une trame multiple, et à activer l'émetteur (28) pour transmettre le message de secours dans la trame libre détectée (34).

21. Unité secondaire selon l'une des revendications 17 à 20,
dans lequel le processeur est réalisé de manière à extraire de la trame de commande de la trame multiple une ou plusieurs trames utiles dans lesquelles le récepteur (22) est prêt à recevoir des données de l'unité centrale, et dans lesquelles l'émetteur (28) est prêt à transmettre des données de l'unité secondaire vers l'unité centrale.

22. Unité secondaire selon la revendication 21,
dans lequel le récepteur (22) et l'émetteur (28) sont désactivés dans les trames utiles dans lesquelles ils ne sont pas prêts.

23. Unité secondaire selon l'une des revendications 18 à 22,
dans lequel, dans la période, l'actionneur est totalement désactivé et le capteur est placé en un mode d'économie, le mode d'économie étant réalisé de sorte que puisse être détectée une situation de secours, toutefois, qu'une consommation de courant soit inférieure à celle dans le cas où sont détectées des données utiles pour une trame utile.

24. Unité secondaire selon l'une des revendications 17 à 23,
dans laquelle la période comporte une pluralité de trames multiples, et dans laquelle le récepteur (22) est désactivé dans la pluralité de trames multiples.

25. Procédé pour communiquer une unité centrale avec une pluralité d'unités secondaires, aux étapes suivantes consistant à :
générer une succession de trames multiples, une trame multiple de la succession de trames multiples présentant une trame de commande (32) et une pluralité de trames utiles (33), la trame de commande d'une trame multiple actuelle (40) présentant des informations sur une association de la pluralité de trames utiles à la pluralité d'unités secondaires et des informations de mode de sommeil pour l'au moins une unité secondaire (BSU1), les informations de mode de sommeil indiquant une période pendant laquelle les unités secondaires doivent être, chacune, en un mode de sommeil, la période comportant au moins une trame multiple (41) qui suit la trame multiple actuelle (40), les informations de mode de sommeil étant contenues exclusivement dans la trame de commande et non pas dans la pluralité de trames utiles ; et
transmettre la succession de trames multiples,
pour l'au moins une unité secondaire étant prédéterminé par l'unité centrale, par les informations de mode de sommeil dans la trame de commande, le temps pendant lequel celle-ci peut fonctionner en mode de sommeil.

26. Procédé pour faire fonctionner une unité secondaire pour communiquer une unité secondaire avec une unité centrale par l'intermédiaire d'une succession de trames multiples, une trame multiple présentant une trame de commande et des trames utiles, aux étapes suivantes consistant à :
recevoir une trame multiple actuelle (40), la trame multiple actuelle présentant une trame de commande et des trames utiles, la trame de commande de la trame multiple actuelle (40) présentant des informations sur une association de la pluralité de trames utiles à la pluralité d'unités secondaires et des informations de mode de sommeil pour une ou plusieurs unités secondaires (BSU1), les informations de mode de sommeil étant prédéterminées par l'unité centrale et indiquant une période pendant laquelle les plusieurs unités secondaires doivent être dans un mode de sommeil, la période comportant au moins une trame multiple (41) qui suit la trame multiple actuelle (40), les informations de mode de sommeil étant contenues exclusivement dans la trame de commande et non pas dans la pluralité de trames utiles ;
interpréter les informations de mode de sommeil contenues dans la trame de commande pour l'unité secondaire (BSU1) et désactiver un récepteur (22) de l'unité secondaire (BSU1) dans la période comportant au moins la trame multiple (41) qui suit la trame multiple actuelle (40).
